(19) Europäisches Patentamt
European Patent Office
Office européen des brevets

(11) Veröffentlichungsnummer: **0 068 299**
**B1**

(12) # EUROPÄISCHE PATENTSCHRIFT

(45) Veröffentlichungstag der Patentschrift: **22.02.84**

(51) Int. Cl.³: **C 07 C 43/13**, C 07 C 43/11, C 07 C 41/46 // C07C41/42, C07C41/03

(21) Anmeldenummer: **82105258.6**

(22) Anmeldetag: **16.06.82**

(54) **Verfahren zur Herstellung lagerstabiler (Poly-)Alkylenglycolmonoalkylether.**

(30) Priorität: **26.06.81 DE 3125107**

(43) Veröffentlichungstag der Anmeldung: **05.01.83 Patentblatt 83/1**

(45) Bekanntmachung des Hinweises auf die Patenterteilung: **22.02.84 Patentblatt 84/8**

(84) Benannte Vertragsstaaten: **BE DE FR GB NL**

(56) Entgegenhaltungen:
**FR - A - 1 194 876**
**GB - A - 958 390**
**GB - A - 1 424 905**
**GB - A - 2 073 188**

(73) Patentinhaber: **BASF Aktiengesellschaft, Carl-Bosch-Strasse 38, D-6700 Ludwigshafen (DE)**

(72) Erfinder: **Bosche, Horst Guenter, Dr., Julius-Leber-Strasse 23, D-6720 Speyer (DE)**
Erfinder: **Nohe, Heinz, Dr., Ruppertsberger Strasse 11, D-6701 Meckenheim (DE)**
Erfinder: **Pachaly, Heinz, Dr., Weinheimer Strasse 15, D-6703 Limburgerhof (DE)**

**EP 0 068 299 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung lagerstabiler (Poly-)Alkylenglycolmonoalkylether

Die vorliegende Erfindung betrifft ein Verfahren zur Herstellung lagerstabiler (Poly-)Alkylenglycolmonoalkylether der allgemeinen Formel I

$$R-(-O-A-)_n-OH \qquad I$$

in der A eine Ethylen- oder 1,2-Propylengruppe bedeutet, R für eine $C_1-C_4$-Alkylgruppe steht und n einen Wert von 1 bis 4 haben kann.

Die definitionsgemäßen Ether des 1,2-Propylenglycols und vor allem des Ethylenglycols finden hauptsächlich als wasserlösliche Lösungsmittel in der Lackindustrie Verwendung und stellen daneben wichtige Zwischenprodukte für organische Synthesen dar.

Die entsprechenden Ether mit zwei oder mehr $(-O-A-)$-Einheiten im Molekül, die man üblicherweise, wenn auch nicht ganz korrekt, als Dialkylenglycolether, Trialkylenglycolether und generell als Polyalkylenglycolether bezeichnet, dienen dagegen im wesentlichen als Hydraulikflüssigkeiten.

Man stellt die Verbindungen I meistens in der Weise her, daß man Ethylenoxid oder 1,2-Propylenoxid bei 100 bis 220°C und 2 bis 50 bar in Gegenwart einer Säure oder insbesondere einer Mineralbase mit einem Alkohol R−OH umsetzt, wobei man je nach den Mengenverhältnissen und den Reaktionsbedingungen Gemische aus den Monoethern des Alkylenglycols (n=1) und der höheren Alkylenglycole (n=2 bis 4) in unterschiedlicher Zusammensetzung erhält. Anschließend trennt man in der Regel den überschüssigen Alkohol R−OH destillativ von diesen Gemischen ab, wonach man sie durch fraktionierte Destillation in ihre Komponenten zerlegt.

Es hat sich nun gezeigt, daß die so erhaltenen Fraktionen nicht lagerstabil sind, denn aus nicht näher bekannten Gründen bilden sich im Laufe der Zeit Säuren, die ihrerseits zu Verfärbungen Anlaß geben, wodurch der Verkaufs- und Gebrauchswert der Produkte I erheblich gemindert wird.

Der Erfindung lag daher die Aufgabe zugrunde, die (Poly-)Alkylenglycolmonoalkylether I gegen die Säurebildung bei der Lagerung zu stabilisieren.

Es wurde nun gefunden, daß die eingangs definierten (Poly-)Alkylenglycolmonoalkylether I gegen die Bildung von Säuren stabil bleiben, wenn man die fraktionierte Destillation der Gemische, die bei der alkalikatalysierten Umsetzung von Ethylenoxid oder 1,2-Propylenoxid mit dem Alkohol R−OH anfallen, in Gegenwart von 0,1 bis 20 ppm Ammoniak, bezogen auf die Menge des eingesetzten Gemisches, vornimmt.

Die Wirkung des Ammoniaks beruht hierbei nicht darauf, daß er zur Neutralisation der entstehenden Säuren dient, denn hierfür sind die Mengen viel zu gering, zumal der Ammoniak im Destillat häufig kaum noch nachweisbar ist. Außerdem bleibt die stabilisierende Wirkung vergleichsweise gering, wenn man die Produkte I erst nach der Destillation mit dem Ammoniak versetzt. Man muß daher annehmen, daß Spuren von störenden Stoffen, welche sonst in das Destillat gelangen und dort als Katalysatoren für die Säurebildung fungieren, vom Ammoniak unter den Bedingungen der Destillation unschädlich gemacht werden.

Die für die fraktionierte Destillation eingesetzten Rohgemische, von denen der überschüssige Alkohol in einer Vorlaufdestillation zweckmäßigerweise bereits abgetrennt ist, setzen sich in der Regel aus 40 bis 90 Gew.-% des Monoalkylenglycolmonoethers, 10 bis 50 Gew.-% des entsprechenden Dialkylenglycolethers und einer Restmenge aus den höheren Alkylenglycolethern zusammen. Außerdem enthalten diese Gemische noch etwa 0,05 bis 0,15 Gew.-% des basischen Katalysators wie NaOH oder KOH. Der Erfolg des erfindungsgemäßen Verfahrens ist weder von Art und Menge des verwendeten basischen Katalysators noch vom Produktspektrum der Alkylenglycolether erkennbar abhängig.

Man nimmt die fraktionierte Destillation der Ethergemische wie üblich bei 2 bis 1000 mbar absolut und 50 bis 200°C kontinuierlich oder diskontinuierlich vor, so daß nähere Angaben sich hierzu erübrigen. Der Ammoniak wird zweckmäßigerweise in den Zulauf der Destillationskolonne gegeben.

Da die Monoalkylenglycolether des Methanols, Ethanols, Propanols, Isopropanols, Butanols Butan-3-ols, Isobutanols und tert.-Butanols die größte wirtschaftliche Bedeutung haben, findet das erfindungsgemäße Verfahren vor allem auf diese Produkte I Anwendung. Ebenso vorteilhaft ist es jedoch auch im Fall der übrigen definitionsgemäßen Produkte I, also solchen, die sich von den höheren Alkylenglycolen (n=2 bis 4) ableiten.

## Beispiel

100 kg eines Ethergemisches, welches durch Umsetzung von Ethylenoxid mit Methanol (Molverhältnis 1 : 8,25) in Gegenwart von 0,01 Gew.-% KOH als Katalysator bei 170°C und 20 bar und anschließende Abtrennung des überschüssigen Methanols gewonnen wurde, wurden mit 0,06 g (=0,6 ppm) Ammoniak versetzt und wie üblich bei 92 bis 170°C und 250 bis 10 mbar (abs.) fraktioniert destilliert.

Als Hauptfraktion (92°C, 250 mbar) fielen 89 kg des Ethylenglycolmonomethylesters an. Eine unmittelbar nach der Destillation entnommene Probe dieser Fraktion wurde mit der dreifachen Menge Wasser verdünnt. Diese Lösung hatte bei Raumtemperatur einen pH-Wert von 6,8.

Eine entsprechende Probe nach zweimonatiger Lagerzeit zeigte einen pH-Wert von 6,5.

Wurde die fraktionierte Destillation unter den gleichen Bedingungen, jedoch ohne den Ammoniak vorgenommen, so fiel der pH-Wert der wäßrigen Probelösung bereits nach einer Woche von anfangs ebenfalls 6,8 auf 4,0.

Der gleiche Befund war im Falle des Ethylenglycolmonoethylethers zu beobachten.

**Patentanspruch**

Verfahren zur Herstellung lagerstabiler (Poly-)-Alkylenglycolmonoalkylether der allgemeinen Formel I

$$R-(-O-A-)_n-OH \qquad I$$

in der A eine Ethylen- oder 1,2-Propylengruppe bedeutet, R für eine $C_1-C_4$-Alkylgruppe steht und n einen Wert von 1 bis 4 haben kann, dadurch gekennzeichnet, daß man die fraktionierte Destillation der Gemische, die bei der alkalikatalysierten Umsetzung von Ethylenoxid oder 1,2-Propylenoxid mit dem Alkohol R—OH anfallen, in Gegenwart von 0,1 bis 20 ppm Ammoniak, bezogen auf die Menge des eingesetzten Gemisches, vornimmt.

**Claim**

A process for the preparation of storage-stable (poly)alkylene glycol monoalkyl ethers of the general formula I

$$R-(-O-A-)_n-OH \qquad I$$

where A denotes an ethylene or 1,2-propylene group, R is alkyl of 1 to 4 carbon atoms, and n may have a value of 1 to 4, wherein the mixture formed in the base-catalyzed reaction of ethylene oxide or 1,2-propylene oxide with the alcohol R—OH is subjected to fractional distillation in the presence of 0.1 to 20 ppm of ammonia, based on the amount of the mixture used.

**Revendication**

Procédé pour la préparation de monoéthers alcoyliques de (poly-)alcoylène-glycols stables à l'entreposage, de formule générale I

$$R-(-O-A-)_n-OH \qquad (I)$$

dans laquelle A représente un groupe éthylène ou 1,2-propylène, R est mis pour un groupe alcoyle en $C_1$ à $C_4$ et n a une valeur de 1 à 4, caractérisé en ce qu'on procède à la distillation fractionnée des mélanges, qui sont obtenus par réaction, avec catalyse basique, d'oxyde d'éthylène ou d'oxyde de 1,2-propylène avec l'alcool R—OH, en présence de 0,1 à 20 ppm d'ammoniac par rapport à la quantité du mélange traité.